# EUROPEAN PATENT SPECIFICATION

(11) **EP 1 496 895 B1**
(45) Date of publication and mention of the grant of the patent: **16.11.2005**
(21) Application number: 03723620.5
(22) Date of filing: 17.03.2003
(51) Int. Cl.: A61K 31/4355, A61P 17/06, A61P 35/00

(54) **USE OF CYCLOPAMINE IN THE TREATMENT OF PSORIASIS AND OTHER SKIN DISORDERS**
VERWENDUNG VON CYCLOPAMINE ZUR BEHANDLUNG VON PSORIASIS UND ANDEREN HAUTERKRANKUNGEN
UTILISATION DE CYCLOPAMINE POUR TRAITER LE PSORIASIS ET AUTRES TROUBLES CUTANES

(30) Priority: 19.04.2002 WO PCT/TR02/00017
(43) Date of publication of application: 19.01.2005
(73) Proprietor: Tas, Sinan, Izmir, 35320 (TR)
(72) Inventor: Tas, Sinan, Izmir 35320 (TR); Avci, Oktay, C Blok, 5-1/12, bornova, Izmir (TR)
(74) Representative: Glas, Holger
(86) International application number: PCT/TR2003/000017
(87) International publication number: WO 2003/088964

(56) References cited:
- WO-A-00/41545
- WO-A-00/74706
- WO-A-01/27135
- WO-A-01/40438
- WO-A-02/07702
- WO-A-99/52534
- WO-A-02/078703
- WO-A-02/078704
- LEBWOHL MARK ET AL: "A randomized, double-blind, placebo-controlled study of clobetasol propionate 0.05% foam in the treatment of nonscalp psoriasis." INTERNATIONAL JOURNAL OF DERMATOLOGY. UNITED STATES MAY 2002, vol. 41, no. 5, May 2002 (2002-05), pages 269-274, XP002251543 ISSN: 0011-9059
- MERK: "The Merk manual" 1999 , MERK RESEARCH LABORATORIES , USA XP002251544 page 817, column 2
- TAIPALE ET AL: "Effects of oncogenic mutations in Smoothened and Patched can be reversed by cyclopamine" NATURE, MACMILLAN JOURNALS LTD. LONDON, GB, vol. 406, no. 6799, 31 August 2000 (2000-08-31), pages 1005-1009, XP002166408 ISSN: 0028-0836

## Description

Cellular differentiation plays a critical role in the generation of multicellular organisms. In human, not only during development but also during the postnatal life, cells in many tissues undergo differentiation to generate new cell types that are required for the proper functioning of organism. Signals for the differentiation of a cell type to another may be derived from the other cellular and non-cellular tissue components in the immediate environment and/or from more distant tissues and organs (e.g. in the form of hormones). Determination of the responses of a cell to the incoming signals is also complex and depends on the nature of the signal or the set of signals as well as on the more intrinsic features of the cell in question (e.g. its differentiation state and the structure of chromatin). During daily life of a human being numerous cells are lost by various mechanisms (because of acute and chronic insults and damage and as a result of the programmed cell death for other reasons). These lost cells are in general replaced by the regulated proliferation and differentiation of their precursors, the progenitor and stem cells. Human skin has relatively greater susceptibility to the environmental insults (e.g. physical trauma) than many internal tissues but is well adapted to the circumstances and displays a constant loss and replacement of its uppermost layer. The dead cells that are shed from the uppermost layer of epidermis are normally replaced by the proliferation of basal layer cells of epidermis and by the differentiation of these cells to generate the suprabasal layers. Abnormalities in this differentiation process, because of environmental and/or genetic reasons, result in various diseases whose skin manifestations are in general readily detected. Skin diseases involving the differentiation disorders of nonepidermal skin cells are also varied. Differences in the underlying causes of these diseases result in their different manifestations and clinical presentations but abnormalities of cellular differentiation is a unifying feature of them. Non-limiting examples of the skin diseases known to display abnormalities of cellular differentiation include ichthyosis vulgaris, lamellar ichthyosis , palmoplantar keratodermas, pityriasis rubra pilaris, Darier disease and several types of skin tumors. Causes of these skin diseases are in general poorly understood. However complexity of the regulation of cellular differentiation and the presence of multiple regulatory factors means that abnormalities of differentiation may sometimes be secondary to the abnormalities that affect primarily other processes. In the absence of a clear understanding of the causes and mechanisms, available treatment strategies for these disease are in general poorly effective (Braun-Falco O. et al (2000) Dermatology, 2^{nd} ed., Springer-Verlag, Berlin).

Cyclopamine is a steroidal alkaloid occurring naturally in the *Veratrum* plants. Teratogenicity of these plants on grazing pregnant animals led to the identification of cyclopamine as an active compound (Keeler R.F. (1969) Phytochemistry 8:223-225). How might have cyclopamine displayed teratogenicity was revealed by the finding that it is an inhibitor of the *hedgehog*/*smoothened* signal transduction (Incardona J.P. et al. (1998) Development 125:3553-3562; Cooper M.K. et al. (1998) Science 280:1603-1607). The *sonic hedgehog* protein has been found to induce differentiation of the precursors of ventral cells in the developing central nervous system (Goodrich L.V. et al. (1998) Neuron 21:1243-1257). WO 0041545, WO 9952534 and WO 0074706 refer to cyclopamine as an inhibitor of the hedgegog pathway, and psoriasis is mentioned among the diseases which can be treated by such inhibitors. Thus inhibition of the *hedgehog*/*smoothened* signaling pathway by cyclopamine in the developing chicken brain was related to the prevention of the formation of ventral cells and, hence, to holopresencephaly (Incardona J.P. et al. (1998) Development 125:3553-3562; Cooper M.K. et al. (1998) Science 280:1603-1607). Holopresencephaly has been known to be the common malformation in the lambs of the sheep grazing *veratrum* (Binns W. et al. (1963) Am J. Vet. Res. 24:1164-1175). Cyclopamine has been reported to inhibit cellular differentiation in other systems as well, including the differentiation of bone marrow cells to erythroid cells (Detmer K. et al. (2000) Dev.Biol. 222:242) and the differentiation of the urogenital sinus to prostate (Berman D.M. et al. (2000) J. Urol. 163:204).

Unanticipated from and contrary to the above summarized teaching of the prior art that cyclopamine acts to inhibit cellular differentiation (Incardona J.P. et al. (1998) Development 125:3553-3562; Cooper M.K. et al. (1998) Science 280:1603-1607;Detmer K. et al. (2000) Dev.Biol.222-242; Berman D.M. et al. (2000) J. Urol. 163:204), we found that cyclopamine rather induced differentiation of the basal cell carcinoma cells that were displaying arrest of the cellular differentiation (Tas S. et al. (2001) PCT/TR01/ 00027, WO 02/078703).Similarly cyclopamine enabled the epidermal cells of psoriatic lesional epidermis to resume normal differentiation (Tas S. et al. (2002) PCT/TR02/00017, WO 02/078704). Again unexpected from the previous reports that cyclopamine had no adverse effect on the viability of transformed cells (Taipale J. et al. (2000) Nature 406: 1005-1009), the cyclopamine-induced differentiation of the basal cell carcinoma cells was accompanied by a massive apoptosis of these cells (Tas S. et al. (2001) PCT/TR01/00027, WO 02/078703).

### SUMMARY OF THE INVENTION:

Cyclopamine is effective in the causations of tumor cell differentiation and apoptosis not only in basal cell carcinomas but also in other skin tumors. Tumors originating from the hair follicle / epidermal cell lineage (e.g. trichoepithelioma) and from melanocytes (e.g. melanocytic nevi) disappeared rapidly upon treatment with cyclopamine. The cyclopamine-induced clearance of psoriatic lesions from the skin of patients with psoriasis is also associated with the causation of cellular differention in lesional epidermis. We disclose that effectiveness of cyclopamine in the treatment of psoriasis is enhanced significantly further by the use of therapeutic compositions comprising of cyclopamine and a corticosteroid and/or by the pre-treatment of lesions with a corticosteroid.

### BRIEF DESCRIPTION OF THE FIGURES:

Fig.1A shows appearance of a psoriatic lesion (~7x8 mm) in the antecubital region of a 29 years old man prior to treatment.
Fig. 1B shows the same lesion as in Fig.1B after treatment with the cyclopamine cream for 24 hours and follow-up without treatment for 44 hours (68^{th} hour from the onset of treatment).
Fig.1C shows a psoriatic lesion (~9x11 mm) in the deltoid region of a 29 years old man prior to treatment.
Fig.1D shows the same lesion as in Fig.1C on the 68^{th} hour of treatment with a pre-mixed cream containing equal volumes of the cyclopamine cream and a cream preparation of clobetasol 17-propionate (0.5 mg/g).
Fig.1E shows a psoriatic lesion (~11x12 mm) in the hypochondrial region of a 29 years old man after 48 hours of treatment with a cream preparation of clobetasol 17-propionate (0.5 mg/g).
Fig.1F shows the same lesion as in Fig.1E on the 24^{th} hour of switching the treatment from clobetasol 17-propionate (0.5 mg/g) to the cyclopamine cream.
Fig.1G shows non-lesional skin tissue with immunohistochemically detected EGFR. The EGFR displaying cells in the epidermis are seen to be restricted to the basal layer. 200X original magnification.
Fig.1H shows non-treated psoriatic lesional skin tissue with immunohistochemically detected EGFR. Suprabasal cells in the lesional epidermis are seen to show markedly increased EGFR expression. 200X original magnification.
Fig. 1l shows psoriatic lesional skin tissue that was treated with the cyclopamine cream for 24 hours before excision and then stained immunohistochemically for EGFR. The EGFR displaying cells in the epidermis are seen to be restricted to the basal layer. 200X original magnification.
Fig.2A shows a trichoepithelioma and a nearby nevus on the cheek of a 82 years old man prior to treatment.
Fig.2B shows the same skin region as in Fig.2A after 24 hours of treatment with cyclopamine.
Fig.2C shows a section from the excised skin region shown in Fig.2B with residual tumor cells. H&E, 400X original magnification.
Fig.2D shows another area from the same tissue as in Fig.2C. In addition to the numerous apoptotic cells and the formation of cystic structures by their removal, the tumor is seen to be infiltrated by mononuclear cells. H&E, 400X original magnification.
Fig.3A shows a pigmented BCC in the lower eyelid of a 59 years old man prior to treatment.
Fig.3B shows the same BCC as in Fig.3A on the third day of treatment with cyclopamine.
Fig.3C shows a section from the treated region of the BCC shown in Fig.3B. H&E, 400X original magnification.
Fig.3D shows close up view of an area of residual tumor cells in a section from the treated region of the BCC shown in Fig.3B. H&E, 400X original magnification.
Fig.3E shows a section from a punch biopsy material obtained from the BCC shown in Fig.3A prior to treatment. H&E, 400X original magnification.
Fig.3F shows a section containing part of the BCC nodule marked by the arrow in Fig.3A.The tissue was excised after 3 days of treatment and 6 days of non-treated follow up. H&E, 400X original magnification.

### COLOR PRINTS

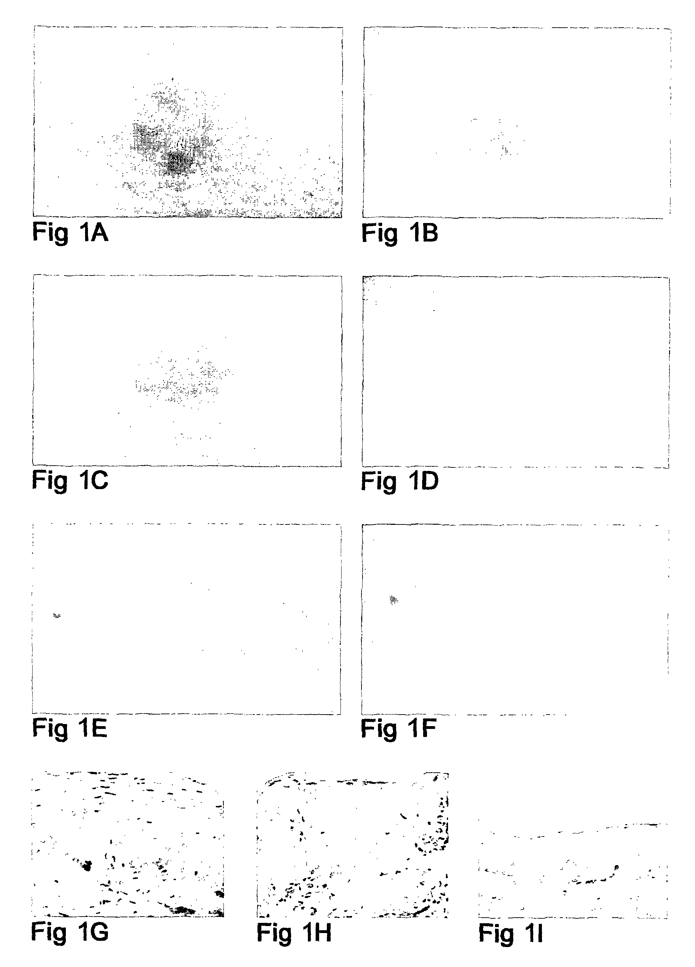

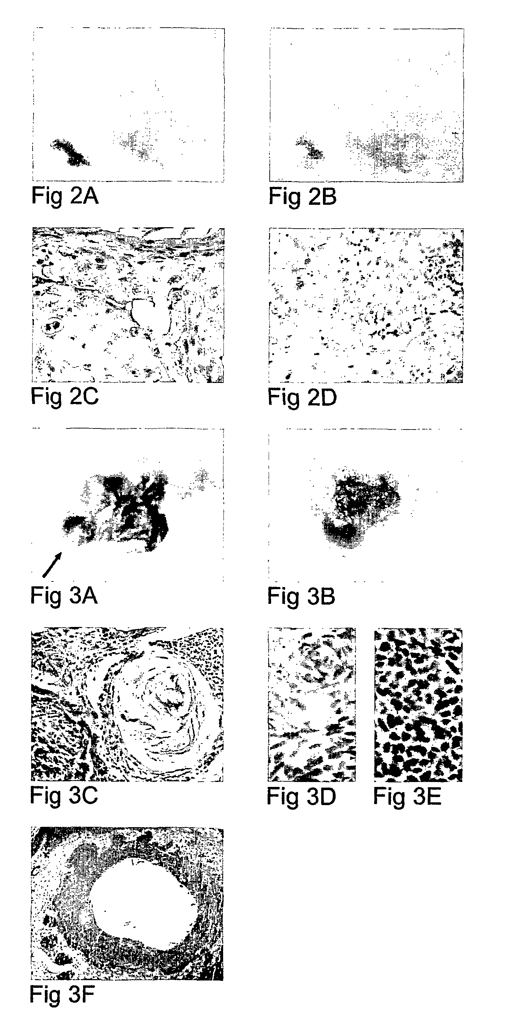

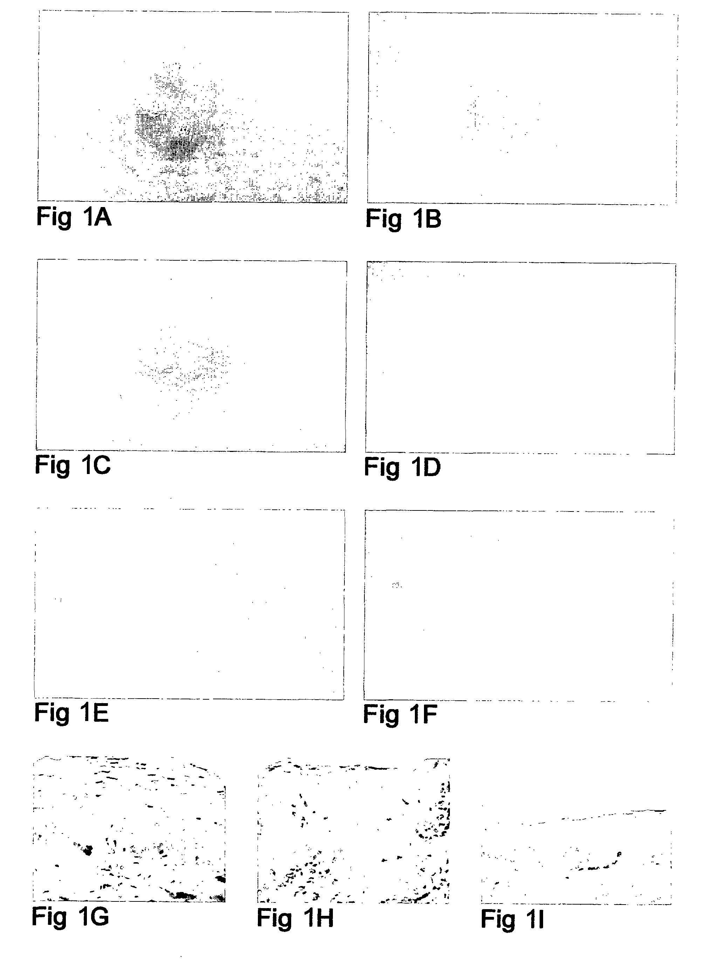

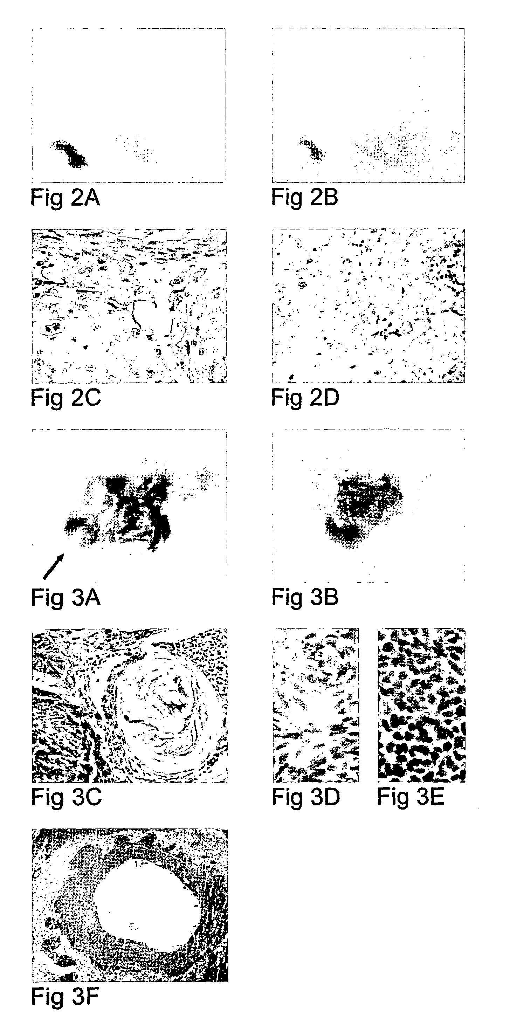

Color prints of the same figures as on pages 14,15 (Fig.1A, Fig.1B, Fig.1C, Fig.1D, Fig.1E, Fig.1F, Fig.1G, Fig.1H, Fig.1 Fig.2A, Fig.2B, Fig.2C, Fig.2D, Fig.3A, Fig.3B, Fig.3C, Fig.3D, Fig.3E, Fig.3F) added as pages 14a, 15a because the immunohistochemical data and findings, due to their nature, can be conveyed best in color rather than in gray-scale; we respectfully request consideration of this fact by the Patent Authority and the keeping of the pages as part of this patent application. However, the pages 14a, 15a may be removed from the patent application if it is deemed necessary by the Patent Authority.

### DETAILED DESCRIPTION OF THE INVENTION:

We disclose that cyclopamine is effective in inducing differentiation of several cell types and that this induction of differentiation can be obtained with sufficient selectivity in vivo (without undue adverse effects) under the conditions we disclose, so that good therapeutic effectiveness can be attained on a number of diseases having the unifying feature of being associated with the inhibition of cellular differentiation. In tumors (both malignant and benign tumors) induction of the differentiation of tumor cells by cyclopamine is disclosed to be associated commonly with the apoptosis (programmed death) of tumor cells, adding thereby to the anti-tumoral therapeutic effectiveness of cyclopamine. The unprecedented therapeutic effectiveness of cyclopamine in psoriasis (disclosed earlier in PCT/TR02/ 00017, WO 02/078704) is increased even further by the use of therapeutic compositions comprising of cyclopamine and a corticosteroid in a suitable pharmaceutical carrier.

Cyclopamine can be dissolved in ethanol or another suitable solvent and can be mixed with a suitable pharmaceutical carrier depending on the desired mode of application. For topical applications, cyclopamine can be dissolved in ethanol or another suitable solvent and can be mixed with a suitable base cream, ointment or gel. Cyclopamine can also be entrapped in hydrogels or in other pharmaceutical forms enabling controlled release and it can be adsorbed onto dermal patches. The effects shown here in figures Fig.1A to Fig.1I and Fig.2A to Fig.2D, and Fig.3A to Fig.3F have been obtained by cream preparations obtained by mixing a solution of cyclopamine in ethanol with a base cream so as to get the indicated final concentrations of cyclopamine. The base cream used is made predominantly of heavy paraffin oil (10% w/w), vaseline (10% w/w), stearyl alcohol (8% w/w), polyoxylsteareth-40 (3% w/w) and water (68% w/w) but another suitably formulated base cream is also possible. Similarities of the lipid versus water solubility characteristics and molecular structures of the cyclopamine and corticosteroids (both being steroidal molecules) facilitate their mixing in the same pharmaceutical carrier. Optimal concentrations of cyclopamine and a corticosteroid in a pharmaceutical form as well as the optimal dosing and application schedules can obviously be affected by such factors as the particular pharmaceutical form, particular corticosteroid and the localization and characteristics of the lesions to be treated; however these can be determined by following well known published methods of optimization. The corticosteroid used in obtaining the effects shown in figures Fig.1C and Fig.1D was clobetasol 17-propionate in a final concentration of ~0.55 mM in base cream (concentration of cyclopamine in the same cream was ~9mM). The corticosteroid used for the pre-treatment of the lesion shown in Fig.1E was clobetasol 17-propionate in a final concentration of ~1.1mM in base cream. Similar corticosteroid actions are obtained by substituting the clobetasol 17- propionate in this cream with ~50 mM hydrocortisone. Other corticosteroid molecules well known in the art are contemplated to be also suitable and capable of replacing the above-mentioned corticosteroid molecules at suitable concentrations (the suitable concentration ranges for a given corticosteroid are also known in the art).

Fig.1A shows a psoriatic plaque on the antecubital region of a 29 years old man prior to treatment. Approximately 20 µl of the cyclopamine cream (18 mM cyclopamine in the base cream described above) was applied onto this lesion every fourth hour for 24 hours. Treatment was then discontinued and the lesion was followed-up. The lesion showed decrease of erythema on the eighth hour of treatment and then continued regression also during the non-treated follow-up to reach to the state shown in Fig.1B on day three and became undetectable on day four.

Fig. 1C shows another psoriatic plaque located on the deltoid region skin of the same patient prior to treatment. Approximately 20 µl of a cream preparation containing -9 mM cyclopamine and ~0.55 mM clobetasol 17 propionate (in base cream) was applied onto this lesion every fourth hour. This lesion also displayed decrease of erythema on the eighth hour and became undetectable on day three (Fig.1D shows its appearance on the 68^{th} hour). In the same patient other psoriatic lesions that were covered with a cream preparation containing ~1.1 mM clobetasol 17-propionate (but no cyclopamine) on every fourth or eight hour showed no detectable change during the same period (i.e. the lesions were persisting on day three). The enhanced therapeutic response to cyclopamine, even at half of the concentration we were using on other lesions of the same patient, prompted further evaluation of the therapeutic compositions comprising of cyclopamine and a corticosteroid. We evaluated in addition pre-treatment of the psoriatic lesions with a topical corticosteroid followed by treatment with the cyclopamine cream (18 mM cyclopamine in base cream). Fig.1E shows a psoriatic plaque on the hypochondrial region of the same patient prior to the applications of cyclopamine. This lesion was treated with the corticosteroid cream alone for 48 hours, corticosteroid was then discontinued and the treatment was switched to the applications of ~20 µl cyclopamine cream (18 mM cyclopamine in base cream) every fourth hour. Fig.1F shows the lesion on the 24^{th} hour of the cyclopamine cream applications and shows near complete disappearance of the lesion by this time.

Severity of psoriatic lesions can be assessed on a semi-quantitative scale by giving separate scores for the erythema, elevation and scaling of a lesion and then by summing up the scores to obtain a score called the EES score of that lesion (Bowman P.H. et al. (2002) J. Am. Acad.Dermatol. 46:907-913). Table I shows comparisons of the therapeutic responses to various forms of treatment, evaluated by the EES scoring. It is seen that use of a therapeutic composition comprising of cyclopamine and a corticosteroid in the treatment of psoriatic lesions enhanced therapeutic effectiveness significantly in comparison to the use of a composition containing only cyclopamine as the active ingredient. Pre-treatment of lesions with corticosteroid for a day, followed by treatment with the cyclopamine cream (18 mM cyclopamine in base cream), enhanced the therapeutic effectiveness similarly. Table I shows that psoriatic lesions treated for a day with a composition containing cyclopamine and then followed up without any treatment also regressed but in general at a relatively slower pace. While this latter property may be of some use in clinical practice, most patients may be expected to prefer the faster clearance of their lesions.

Cyclopamine cream alone caused regression and clearance of psoriatic lesions at paces much faster than those obtained by conventional treatments as described earlier (PCT/TR 02/00017, WO 02/078704) and in here. However further enhancement of the therapeutic response was possible when cyclopamine was used together with, or after a brief pre-treatment with, corticosteroids. This property increases the clinical utility of cyclopamine treatment and may in part be related to the anti-inflammatory effects of corticosteroids. Contributions of the inflammatory cells and cytokines to the development of psoriatic lesions have been well-recognized (Krueger J.G. et al. (1990) J. Invest. Dermatol. 94:135S-140S). Expressions of both the epidermal growth factor receptor (EGFR) and of one or more of its ligands (e.g. the transforming growth factor alpha) are known to be markedly increased in psoriatic lesional epidermis. These may set up an autocrine stimulation loop contributed further by the causation of increase of epidermal EGFR by inflammation (Krueger J.G. et al. (1990) J. Invest. Dermatol. 94:135S-140S). However, unusually rapid therapeutic response to the cyclopamine cream alone (but not to the corticosteroid alone) and, indeed, continued regression of the psoriatic lesions after discontinuation of cyclopamine applications are consistent with the effective intervention by cyclopamine (but not by corticosteroids) with a key/proximal pathogenic events. Because EGFR over expression in the suprabasal layers of psoriatic lesional epidermis may set up an autocrine stimulation loop and since return of EGFR expression to the basal layer of epidermis is regarded to be one of the first signs of effective treatment of psoriasis by various modalities (King L.E.Jr et al. (1990) J. Invest.Dermatol. 95:10S-12S), response of the EGFR expression pattern to cyclopamine treatment was evaluated. For this purpose mouse monoclonal antibody EGFR.113 (Novocastra Labs. Ltd., U.K.) against the human EGFR was used as primary antibody and the immunohistochemical assay was performed as recommended by the manufacturer. Fig. 1I shows that application of the cyclopamine cream onto psoriatic lesions caused rapid normalization of the EGFR expression pattern and that the immunohistochemically detected EGFR became again restricted mostly to the basal layer of epidermis by the 24^{th} hour of treatment (Fig.11). The EGFR expression pattern following the cyclopamine treatment of lesion (Fig. 1I) was indistinguishable from that seen in non-lesional skin (Fig.1G). Psoriatic lesional skin treated with the placebo cream (i.e. the base cream lacking cyclopamine), on the other hand, continued to display the markedly contrasting pathological pattern and overexpression of EGFR in the suprabasal layers of epidermis (Fig.1H). Thus cyclopamine treatment had rapid therapeutic effect on the psoriatic lesions also by the criterion of EGFR expression (King L.E.Jr et al.(1990) J.Invest.Dermatol. 95:10S-12S).

Causation of the differentiation of the basal cell carcinoma cells by cyclopamine has been found associated with their apoptosis (programmed death) (PCT/TR 01/00027, WO 02/078703). Figures Fig. 2A to Fig. 2D show that such effects of cyclopamine are not restricted to the basal cell carcinomas. Trichoepithelioma is a tumor associated with genetic changes that cause increased *hedgehog-smoothened* signaling (Vorechovsky I et al. (1997) Cancer Res. 57:4677-4681; Nilsson M. et al. (2000) Proc. Natl. Acad. Sci. USA 97:3438-3443). Fig 2A shows a trichoepithelioma on the cheek of 82 years old man prior to treatment and Fig 2B shows the same skin area after only 24 hours of exposure to the cyclopamine cream (18 mM cyclopamine in the base cream; -25 µl cream was applied every third hour directly onto the tumor). Because of the rapid regression, treatment was discontinued on the 24^{th} hour and the entire skin area corresponding to the original tumor was excised for investigation. Fig 2C and Fig 2D show the tissue region that contained residual tumor cells on the 24^{th} hour and reveal marked apoptotic activity among these residual tumor cells. Cystic space resulting from the apoptotic removal of tumor cells (Fig 2C, Fig 2D) as well as the mononuclear cellular infiltration of tumor (Fig 2D) are seen. Another noteworthy finding in this patient was the decreased size and pigmentation of a mole located nearby the treated tumor on the 24^{th} hour of treatment (Fig 2B versus Fig 2A). As cyclopamine could have diffused from the adjacent area of application, the mole (a benign melanocytic tumor) appears to be sensitive to relatively low concentrations of cyclopamine. Indeed treatment of melanocytic nevi with the cyclopamine cream (18 mM cyclopamine in base cream) in another volunteer also caused similarly rapid depigmentation and disappearance of the nevi (data not shown).

Fig 3A shows a pigmented basal cell carcinoma (BCC) on the lower eyelid of a 59 years old man prior to treatment. Cyclopamine cream (18 mM cyclopamine in base cream) was applied in this patient onto all of the nodules except for the one marked by the arrow. This nodule, which could have received cyclopamine only by diffusion from the adjacent treated region, would be exposed to a relatively lower concentration of cyclopamine. As the pigmented nature of this tumor facilitated clinical follow-up, treatment (application of -20 µl cyclopamine cream on every fourth hour) was discontinued on the third day when the tumor in the treated region had largely regressed but still contained visible parts (Fig. 3B). The tumor was then followed up without treatment for a study of the possible late effects. A clear further clinical regression was not observed in the absence of treatment and the skin area corresponding to the original tumor was excised on the sixth day of follow up (ninth day from the start of treatment). Hematoxylene-eosine stained sections from the treated region of tumor revealed many cystic spaces that lacked tumor cells (Fig.3C).The absence of an epithelium lining these cysts (Fig.3C) is consistent with the representation by these cysts of the tissue areas that were formerly occupied by the tumor cells. At this time point (the sixth day of non-treated follow up), tissue sections displayed a relative paucity of the apoptotic cells (Fig.3C) consistent with the known rapidity of the clearance of apoptotic cells from live tissues. On the other hand the residual tumor cells, particularly near the edges of cysts, showed unusually high frequencies of cells displaying features of spinous differentiation (e.g. the area towards the lower left of Fig.3C; seen more clearly on higher magnification as exemplified from another area in Fig.3D). Similar areas of differentiation or cysts were absent in the punch biopsy material obtained from the same tumor prior to the initiation of treatment (Fig.3E). The tumor nodule that received relatively lower concentration of cyclopamine (marked by arrow in Fig.3A) had a large cystic center on the sixth day of follow up (Fig.3F). The residual periphery of the nodule, however, continued to have cells with typical BCC morphology although frequency of the cells with features of differentiation (e.g. with enlarged and more eosinophilic cytoplasm) was again increased and smaller cysts existed within this periphery (Fig.3F). Thus, while the tumor response to optimal concentrations of cyclopamine was relatively rapid, exposure to suboptimal concentrations left behind tumor cells that persisted during the period of follow up.

These examples further illustrate effectiveness of the described treatment in causations of tumor cell differentiation and apoptosis and in obtaining rapid clinical regression of the tumors that display increased hedgehog-smoothened signaling. Effectiveness on several independent tumors in unrelated patients with differing genotypes is consistent with the general utility of the described treatment. Treatment with cyclopamine under the described conditions has not revealed undue adverse effects on normal tissue components (including the putative stem cells) by histological /immunohistochemical criteria as we have disclosed also earlier (PCT/TR01/00027, WO 02/078704). Furthermore former skin sites of cyclopamine application that have been followed up to more than 2 years at the time of this writing continue to display healthy-looking normal skin and hair suggesting functional preservation of the stem cells and long-term safety.

Most of the biological and therapeutic effects of cyclopamine disclosed here and earlier (WO 02/078703, WO 02/078704) have a unifying feature in that they are related to the causation of cellular differentiation. It is therefore specifically contemplated that other molecules can be derived from cyclopamine or synthesized in such a way that they exert similar receptor binding properties and biological and therapeutic effects as cyclopamine. Such molecules are called here as "derivatives of cyclopamine". The term "derivatives of cyclopamine ", as used here, is defined as follows:

A molecule that contains the region of the cyclopamine molecule involved in the binding of cyclopamine to its biological target but contains in addition modifications of the parent cyclopamine molecule in such ways that the newly derived molecule continues to be able to bind specifically to the same biological target (i.e. the smoothened protein) to exert the biological effects of cyclopamine disclosed in this invention and in WO 02/078703 and WO 02/078704. Such modifications of cyclopamine may include one or more permissible replacement of or deletion of a molecular group in the cyclopamine molecule or in addition of a molecular group (particularly a small molecular group such as the methyl group) to the cyclopamine molecule provided that the resultant molecule is stable and possesses the capability of specific binding to the same biological target as cyclopamine to exert the biological effects of cyclopamine disclosed in this invention and in WO 02/078703 and WO 02/078704. Derivation of such new molecules from cyclopamine can be readily achieved by those skilled in the art and the continuance or abolishment of the possession of the biological effects of cyclopamine in the newly derived molecule can also be readily determined by those skilled in the art, for example by testing for the biological effects disclosed in this application and in WO 02/078703 and WO 02/078704.

**Table I.**

| Semi-quantitative Evaluation Of The Responses Of Psoriatic Lesions To Different Types Of Treatment | | | |
|---|---|---|---|
| | | ESS score* of the psoriatic lesions before and on day 4 of treatment (mean ± S.D.) | |
| Treatment | Number of Lesions That were scored | Pre-Treatment | Day 4 |
| Base Cream | 5 | 6.6 ± 1.0 | 6.0 ± 0.6 |
| Cyclopamine Cream | 4 | 6.5 ± 0.4 | 0.4 ± 0.2 |
| (Cyclopamine+CS) Cream | 5 | 6.7 ± 0.9 | 0 ± 0 |
| Cyclopamine Cream, 1 day only | 6 | 6.8 ± 1.7 | 0.9 ± 0.3 |
| (Cyclopamine+CS) Cream, 1 day only | 7 | 6.7 ± 1.3 | 0.4 ± 0.3 |

| | | | |
|---|---|---|---|
| *Sum of the erythema, elevation and scaling scores (each on a 0 to 4 scale with 0.5 increments as described by Bowman P.H. et al (2002) J. Am. Acad. Dermatol. 46:907-913). In the "1 day only" treatments, treatment with cyclopamine was continued for 1 day only. The lesions were then followed up without treatment and the EES scores were determined. Numbers of lesions scored in each treatment category are shown. Scores of the lesions that were excised before day 4 for histopathological /immunohistochemical analyses are not included in this evaluation and calculations. (Cyclopamine+CS) cream refers to treatment with a cream preparation containing ~ 9 mM cyclopamine and ~ 0.55 mM clobetasol 17-propionate or to treatment with a cream preparation containing ~ 18 mM cyclopamine after a day of pre-treatment with a cream preparation containing ~ 1.1 mM clobetasol 17-propionate. Results of these two types of treatment were similar and were therefore calculated as a single group. | | | |

## Claims

1. Use of cyclopamine or a pharmaceutically acceptable salt or derivative thereof, and a corticosteroid, for the manufacture of a pharmaceutical composition for treatment of skin lesions associated with impaired differentiation of epidermal cells.

2. Use according to claim 1, wherein the corticosteroid is clobetasol-17-propionate or hydrocortisone.

3. Use according to anyone of claims 1 or 2, wherein the skin lesions are psoriatic.

4. A pharmaceutical composition comprising cyclopamine or a pharmaceutically acceptable salt or derivative thereof, and a corticosteroid.

5. The pharmaceutical composition according to claim 4, wherein the corticosteroid is clobetasol-17-propionate or hydrocortisone.

6. The pharmaceutical composition according to any one of claims 4 or 5, wherein the composition is in the form of a cream, ointment, gel, hydrogel, a pharmaceutical form enabling controlled release, or a solution in ethanol or another suitable solvent, or it is adsorbed onto a dermal patch.

7. The pharmaceutical composition according to claim 4, comprising cyclopamine or a pharmaceutically acceptable salt or derivative thereof in a concentration of 10 mM to 20 mM, and clobetasol-17-propionate in a concentration of 0,5 mg/g to 1,0 mg/g or hydrocortisone in a concentration of 10 mM to 50 mM.

8. Use of cyclopamine or a pharmaceutically acceptable salt or derivative thereof, for the manufacture of a pharmaceutical composition for the treatment of skin disorders being associated with impaired cellular differentiation selected from the group consisting of melanocytic tumors, ichthyosis vulgaris, lamellar ichthyosis, palmoplantar keratodermas, pityriasis rubra pilaris, and Darier disease.

9. Use according to claim 8, wherein the melanocytic tumor is a nevus.

10. Use of cyclopamine or a pharmaceutically acceptable salt or derivative thereof, for the manufacture of a pharmaceutical composition for application on patients having skin disorders associated with increased pigmentation.

## Patentansprüche

1. Verwendung von Cyclopamin oder eines pharmazeutisch akzeptablen Salzes oder Derivates davon, sowie eines Corticosteroids, für die Herstellung einer pharmazeutischen Zusammensetzung für die Behandlung von Hautläsionen verbunden mit beeinträchtigter Differenzierung epidermaler Zellen.

2. Verwendung nach Anspruch 1, wobei das Corticosteroid Clobetasol-17-Propionat oder Hydrocortison ist.

3. Verwendung nach einem der Ansprüche 1 oder 2, wobei die Hautläsionen psoriatisch sind.

4. Pharmazeutische Zusammensetzung umfassend Cyclopamin oder ein pharmazeutisch akzeptables Salz oder Derivat davon, sowie ein Corticosteroid.

5. Pharmazeutische Zusammensetzung nach Anspruch 4, wobei das Corticosteroid Clobetasol-17-Propionat oder Hydrocortison ist.

6. Pharmazeutische Zusammensetzung nach einem der Ansprüche 4 oder 5, wobei die Zusammensetzung in Form einer Creme, Salbe, eines Gels, Hydrogels, einer pharmazeutischen Form mit kontrollierter Freisetzung, oder einer Lösung in Ethanol oder einem anderen geeigneten Lösungsmittel vorliegt oder auf einem Pflaster für die Haut adsorbiert ist.

7. Pharmazeutische Zusammensetzung nach Anspruch 4, umfassend Cyclopamin oder ein pharmazeutisch akzeptables Salz oder Derivat davon in einer Konzentration von 10 mM bis 20 mM sowie Clobetasol-17-Propionat in einer Konzentration von 0,5 mg/g bis 1,0 mg/g oder Hydrocortison in einer Konzentration von 10 mM bis 50 mM.

8. Verwendung von Cyclopamin oder eines pharmazeutisch akzeptablen Salzes oder Derivats davon, für die Herstellung einer pharmazeutischen Zusammensetzung für die Behandlung von Hautkrankheiten die mit beeinträchtigter zellulärer Differenzierung verbunden sind, ausgewählt aus der Gruppe bestehend aus Melanocyten-Tumoren, Ichthyosis Vulgaris, Lamellarichthyosis, palmoplantare Keratodermas, Pityriasis Rubra Pilaris, und Morbus Darier.

9. Verwendung nach Anspruch 8, wobei der Melanocyten-Tumor ein Nevus ist.

10. Verwendung von Cyclopamin oder eines pharmazeutisch akzeptablen Salzes oder Derivats davon, für die Herstellung einer pharmazeutischen Zusammensetzung für die Anwendung bei Patienten mit Hautkrankheiten die mit erhöhter Pigmentierung verbunden sind.

## Revendications

1. Utilisation de cyclopamine ou d'un sel ou d'un dérivé pharmaceutiquement acceptable de ladite substance, pour la fabrication d'une composition pharmaceutique pour traiter les lésions cutanées associées à un trouble de la différenciation des cellules épidermiques.

2. Utilisation selon la revendication 1, dans laquelle le corticostéroïde est du clobetasol-17-propionate ou de l'hydrocortisone.

3. Utilisation selon l'une quelconque des revendications 1 ou 2, dans laquelle les lésions cutanées sont psoriasiques.

4. Composition pharmaceutique comprenant de la cyclopamine ou un sel ou un dérivé pharmaceutiquement acceptable de ladite substance, et un corticostéroïde.

5. Composition pharmaceutique selon la revendication 4, dans laquelle le corticostéroïde est du clobetasol-17-propionate ou de l'hydrocortisone.

6. Composition pharmaceutique selon l'une quelconque des revendications 4 ou 5, dans laquelle la composition prend la forme d'une crème, d'un onguent, d'un gel, d'un hydrogel, d'une forme pharmaceutique permettant une libération contrôlée, ou d'une solution d'éthanol ou d'un autre solvant approprié, ou qui est adsorbé sur un patch dermique.

7. Composition pharmaceutique selon la revendication 4, comprenant de la cyclopamine ou un sel ou un dérivé pharmaceutiquement acceptable de ladite substance dans une concentration de 10 mM à 20 mM, et du clobetasol-17-propionate dans une concentration de 0,5 mg/g à 1,0 mg/g ou de l'hydrocortisone dans une concentration de 10 mM à 50 mM.

8. Utilisation de cyclopamine ou d'un sel ou d'un dérivé pharmaceutiquement acceptable de ladite substance, pour la fabrication d'une composition pharmaceutique pour le traitement des troubles cutanés étant associés à un trouble de la différenciation cellulaire choisi parmi le groupe constitué par des tumeurs mélanocytaires, ichtyose vulgaire, ichtyose lamellaire, kératodermie palmoplantaire, Pilaris de rubra de Pityriasis («pyriasis rubra pilaris ») et maladie de Darier.

9. Utilisation selon la revendication 8, dans laquelle la tumeur mélanocytaire est un naevus.

10. Utilisation de cyclopamine ou d'un sel ou d'un dérivé pharmaceutiquement acceptable de ladite substance, pour la fabrication d'une composition pharmaceutique destinée à être appliquée sur des patients présentant des troubles cutanés associés à une pigmentation accrue.
